# EUROPEAN PATENT SPECIFICATION

(11) **EP 3 096 749 B1**
(45) Date of publication and mention of the grant of the patent: **15.05.2019**
(21) Application number: 15702348.2
(22) Date of filing: 21.01.2015
(51) Int. Cl.: A61K 31/137, A61K 31/365, A61K 31/4035, A61K 31/7048, A61K 45/06, A61P 3/04, A61K 31/37, A61K 31/375, A61K 31/55

(54) **METHODS FOR THE TREATMENT OF OBESITY USING APREMILAST**
VERFAHREN ZUR BEHANDLUNG VON ADIPOSITAS MIT APREMILAST
MÉTHODES DE TRAITEMENT DE L'OBÉSITÉ À L'AIDE D'APRÉMILAST

(30) Priority: 24.01.2014 US 201461931143 P
(43) Date of publication of application: 30.11.2016
(73) Proprietor: Celgene Corporation, Summit, NJ 07901 (US)
(72) Inventor: HOUGH, Douglas, R, Martinsville, NJ 08836 (US); STEVENS, Randall, M., Rockport, MA 01966 (US)
(74) Representative: Jones Day
(86) International application number: PCT/US2015/012201
(87) International publication number: WO 2015/112568

(56) References cited:
- WO-A1-2012/149251
- US-A1- 2006 148 882
- US-A1- 2013 310 369
- FIELD: "Roflumilast, a Novel Phosphodiesterase 4 Inhibitor, for COPD Patients with a History of Exacerbations", CLINICAL MEDICINE INSIGHTS: CIRCULATORY, RESPIRATORY AND PULMONARY MEDICINE, vol. 2011:5, 1 October 2011 (2011-10-01), pages 57-70, XP055172392, DOI: 10.4137/CCRPM.S7049
- JENSTERLE, M. ET AL.: "Phosphodiesterase 4 inhibition as a potential new therapeutic target in obese women with polycystic ovary syndrome", J OF CLINICAL ENDOCRINOLOGY AND METABOLISM, vol. 99, no. 8, 13 May 2014 (2014-05-13), pages E1476-E1481, XP009182853, DOI: 10.1210/jc.2014-1430

## Description

### 2. FIELD

Provided herein is apremilast for use in methods for treating, preventing and/or managing obesity by the administration of apremilast, alone or in combination with other therapeutics or treatment regimens. Also provided herein are pharmaceutical compositions and dosage forms comprising specific amounts of apremilast suitable for use in methods of treating, preventing and/or managing obesity.

### 3. BACKGROUND

Obesity is a substantial public health crisis in the United States and internationally, with the prevalence increasing rapidly in numerous nations. It was reported that the prevalence of obesity among American men and women was almost 36% in 2009 and 2010. *See* Flegal KM et al., "Prevalence of obesity and trends in the distribution of body mass index among US adults, 1999-2010" JAMA, 2012, 307(5):491-7. Obesity represents a state of excess storage of body fat. The term overweight is defined as an excess of body weight for height. Normal, healthy men have a body fat percentage of 15-20%, while normal, healthy women have a percentage of approximately 25-30%. *See* Gallagher D et al., "Healthy percentage body fat ranges: an approach for developing guidelines based on body mass index" Am J Clin Nutr., 2000, 72(3):694-701. Because differences in weight among individuals are only partly the result of variations in body fat, body weight is a limited index of obesity. The body mass index (BMI) is used far more commonly than body fat percentage to define obesity.

The most widely accepted classifications for degrees of obesity are those from the World Health Organization (WHO), based on body mass index (BMI). The WHO designations are as follows:
Grade 1 overweight (commonly and simply called overweight): BMI of 25-29.9 kg/m²
Grade 2 overweight (commonly called obesity): BMI of 30-39.9 kg/m²
Grade 3 overweight (commonly called severe or morbid obesity): BMI ≥40 kg/m².

The effective approaches for weight loss include lifestyle changes, such as diet, physical activity, and behavioral counseling. Pharmacotherapy is recommended for individuals who are unable to achieve weight loss with lifestyle interventions alone. Nainggolan L, "New obesity guidelines: authoritative 'roadmap' to treatment" Medscape Medical News, Nov. 2013 (available at http://www.medscape.com/viewarticle/814202). Currently, drugs used to manage obesity include centrally acting medications that impair dietary intake, medications that act peripherally to impair dietary absorption, and medications that increase energy expenditure.

US 2006/0079540 A1 discloses the use of a PDE4 inhibitor for the treatment of cachexia. The clinical picture of cachexia includes weight loss. However, the use of PDE4 inhibitor disclosed in the publication was not for the treatment of obesity with the intent to induce weight loss.

Weight decrease was observed in treatment of chronic obstructive pulmonary disease (COPD) with roflumilast as a side effect. *See* Klaus F. Rabe, British Journal of Pharmacology, 2011, 163:53-67; Stephen K. Field, Clinical Medicine Insights: Circulatory, Respiratory and Pulmonary Medicine, 2011, 5:57-70; Kumar et al., BMC Medicine, 2013, 11:96:1-8; and Nicola J. Sinden et al., Ther Adv Chronic Dis, 2010, 1(2):43-57. Weight loss is frequently seen in COPD patients particularly when their disease worsens as they often have difficulty eating when out of breath. *Id.*

Unlike weight decrease observed as a side effect in COPD patients treated with another PDE4 inhibitor, apremilast in the present invention provides benefit of weight loss to patients with a mean BMI of 30 or more, which is the clinical definition of obesity.

### 4. SUMMARY

Provided herein is apremilast or a pharmaceutically acceptable polymorph, salt or solvate thereof for use in methods for treating, preventing and/or managing obesity or overweight in humans in need thereof. The invention comprises administering to a patient in need of such treatment, prevention or management with a therapeutically or prophylactically effective amount of apremilast, or a pharmaceutically acceptable metabolite, polymorph, salt, solvate (*e.g*., hydrate) or clathrate thereof.

In some embodiments, provided herein is stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt or solvate thereof for use in a method of treating, preventing and/or managing obesity or overweight, which comprises orally administering to a patient having obesity or overweight an effective amount of stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt, or solvate thereof.

In some embodiments, the patient is administered about 10 mg twice daily (BID) of stereomerically pure (+)-2-[1-(3-ethoxy-4-methoxyphenyl)-2-methylsulfonylethy1]-4-acetylaminoisoindoline-1,3-dione, or a pharmaceutically acceptable polymorph, salt, or solvate thereof. In some embodiments, the dose is about 20 mg BID. In other embodiments, the dose is about 30 mg BID. In some embodiments, the dose is about 40 mg BID or 80 mg once daily (QD).

In some embodiments, stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt, or solvate thereof is administered orally in a dosage form such as a tablet and a capsule.

In some embodiments, the invention further comprises the administration of a therapeutically or prophylactically effective amount of one or more second active agents. Examples of the second active agents, include but are not limited to, phentiramine, lorcaserin, topiramate, orlistat or other approved weight loss medications. In some embodiments, the second active agents, include but are not limited to, an analgesic, a non-steroidal anti-inflammatory drug (*i.e.,* NSAID), an anti-inflammatory agent, a COX-2 inhibitor, an opioid, a corticosteroid, a biologic agent, and an immunosuppressant.

In some embodiments, the invention further comprises other weight reduction measures, such as change in dietary habits, exercise programs, or psychological support programs.

### 5. DETAILED DESCRIPTION

### 5.1 Definitions

As used herein, the term "apremilast" refers to (+)-2-[1-(3-ethoxy-4-methoxyphenyl)-2-methylsulfonylethyl]-4-acetylaminoisoindoline-1,3-dione, also known as N-[2-[(1S)-1-(3-ethoxy-4-methoxylphenyl)-2-(methylsulfonyl)ethyl]-2,3-dihydro-1,3-dioxo-1H-isoindol-4-yl]acetamide. Apremilast has the following structure:

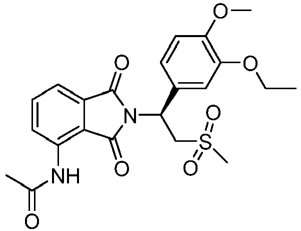

Without being limited by theory or mechanism of action, Apremilast is an inhibitor of phosphodiesterase 4 (PDE4), and works intracellularly to modulate a network of pro- and anti-inflammatory mediators. Phosphodiesterase 4 is a cyclic adenosine monophosphate (cAMP)-specific phosphodiesterase (PDE) and the dominant PDE in inflammatory cells. Inhibition of PDE4 elevates intracellular cAMP levels, which in turn down regulates the inflammatory response by modulating the expression of tumor necrosis factor-alpha (TNF-α), interleukin (IL)-23, and other inflammatory cytokines. Elevation of cAMP also increases anti inflammatory cytokines such as IL-10. These pro- and anti-inflammatory mediators have been implicated in psoriasis and psoriatic arthritis. *See, e.g.,* Schafer et al., "Apremilast, a cAMP phosphodiesterase-4 inhibitor, demonstrates anti-inflammatory activity in vitro and in a model of psoriasis," Br. J. Pharmacol., 2010,159(4):842-55.

Adipocyte-specific lipin 1 and mitochondrial dysfunction may be involved in the mechanism of weight loss, by which apremilast may increase lipogenesis and baseline metabolic activity, through PDE4 inhibition and elevation of Protein Kinase A and activation of the CREB (cAMP Responsive Element Binding Protein) pathway. *See* Aurelia De Pauw et al., The American Journal of Pathology, September 2009, Vol. 175, No. 3:927-939; and Mayurranjan S. Mitra et al., PNAS, 2013, Vol. 110, No. 2:642-647.

Apremilast is under clinical development for the treatment of adult inflammatory autoimmune disorders that involve elevated cytokine levels, such as psoriasis, psoriatic arthritis, rheumatoid arthritis, Behçet's disease and ankylosing spondylitis.

As used herein and unless otherwise indicated, the term "pharmaceutically acceptable salt" includes, but is not limited to, salts prepared from pharmaceutically acceptable non-toxic acids or bases including inorganic acids and bases and organic acids and bases. Suitable pharmaceutically acceptable base addition salts provided herein include metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts.

As used herein and unless otherwise indicated, the term "hydrate" means a compound provided herein or a salt thereof, that further includes a stoichiometric or non-stoichiometric amount of water bound by non-covalent intermolecular forces.

As used herein and unless otherwise indicated, the term "solvate" means a solvate formed from the association of one or more solvent molecules to a compound provided herein. The term "solvate" includes hydrates (*e.g*., mono-hydrate, dihydrate, trihydrate, tetrahydrate and the like).

As used herein and unless otherwise indicated, the term "polymorph" means solid crystalline forms of a compound provided herein or complex thereof. Different polymorphs of the same compound can exhibit different physical, chemical, pharmacological and /or spectroscopic properties.

As used herein and unless otherwise specified, the term "prodrug" means a derivative of a compound that can hydrolyze, oxidize, or otherwise react under biological conditions (*in vitro, ex vivo* or *in vivo*) to provide the compound. Examples of prodrugs include, but are not limited to, derivatives and metabolites of apremilast that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Prodrugs can typically be prepared using well-known methods, such as those described by 1 Burger's Medicinal Chemistry and Drug Discovery, 172-178, 949-982 (Manfred E. Wolff ed., 5th ed. 1995).

As used herein, and unless otherwise specified, the term "enantiomer," "isomer" or "stereoisomer" encompasses all enantiomerically/stereomerically pure and enantiomerically/stereomerically enriched compounds provided herein.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure, when the compound contains greater than or equal to 80%, 85%, 90%, 95%, 96%, 97%, 98% or 99% of one stereoisomer, and 20%, 15%, 10%, 5%, 4%, 3%, 2%, 1% or less of the counter stereoisomer. "Substantially free of its (R) enantiomer" is encompassed by the term stereomerically pure or enantiomerically pure.

As used herein, term "adverse effect" includes, but is not limited to gastrointestinal, renal and hepatic toxicities, leukopenia, increases in bleeding times due to, *e.g*., thrombocytopenia, and prolongation of gestation, nausea, vomiting, somnolence, asthenia, dizziness, teratogenicity, extra-pyramidal symptoms, akathisia, cardiotoxicity including cardiovascular disturbances, inflammation, male sexual dysfunction, and elevated serum liver enzyme levels. The term "gastrointestinal toxicities" includes but is not limited to gastric and intestinal ulcerations and erosions. The term "renal toxicities" includes but is not limited to such conditions as papillary necrosis and chronic interstitial nephritis.

As used herein, the term "patient" refers to a mammal, particularly a human. In some embodiments, the patient is a female. In further embodiments, the patient is a male. In further embodiments, the patient is a child or adolescent.

As used herein, and unless otherwise specified, the terms "treat," "treating" and "treatment" contemplate an action that occurs while a patient is suffering from the specified disease, disorder, or condition which reduces the severity or symptoms of the disease, disorder, or condition, or retards or slows the progression of symptoms of the disease, disorder, or condition.

As used herein, unless otherwise specified, the terms "prevent," "preventing" and "prevention" contemplate an action that occurs before a patient begins to suffer from the specified disease, disorder, or condition, which inhibits or reduces the severity or symptoms of the disease, disorder, or condition.

As used herein, and unless otherwise indicated, the terms "manage," "managing," and "management" encompass preventing the recurrence of the specified disease, disorder, or condition in a patient who has already suffered from the disease, disorder, or condition, and/or lengthening the time that a patient who has suffered from the disease, disorder, or condition remains in remission. The terms encompass modulating the threshold, development and/or duration of the disease, disorder, or condition, or changing the way that a patient responds to the disease, disorder, or condition.

### 5.2 Compounds for Use in Methods of Treatment

Provided herein are compounds for use in methods of treating, managing and/or preventing obesity or overweight, which comprise administering to a patient in need of such treatment, management or prevention a therapeutically or prophylactically effective amount of apremilast, or a pharmaceutically acceptable metabolite, polymorph, salt, solvate or clathrate thereof.

In some embodiments, the invention also encompasses inhibiting or averting symptoms of obesity as well as addressing the disease itself, prior to the onset of symptoms by administering apremilast, or a pharmaceutically acceptable metabolite, polymorph, salt, solvate or clathrate thereof.

In some embodiments, provided herein is stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt or solvate thereof for use in a method of treating obesity or decreasing weight, which comprises orally administering to a patient having obesity or overweight an effective treatment amount of stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt, or solvate thereof.

In certain embodiments, apremilast is orally administered to a patient having obesity or overweight in a dose of about 10 mg, 20 mg, 30 mg, or 40 mg twice daily. In certain embodiments, apremilast may be used alone in doses of 20 mg, 30 mg, or 40 mg twice daily (BID), or in combination with other weight reduction measures, such as change in dietary habits, exercise programs, or psychological support programs. The weight loss may be observed within a few months of administration and may progress with continued use.

In some embodiments, the patient is administered about 10 mg BID of stereomerically pure (+)-2-[1-(3-ethoxy-4-methoxyphenyl)-2-methylsulfonylethy1]-4-acetylaminoisoindoline-1,3-dione, or a pharmaceutically acceptable polymorph, salt, or solvate thereof. In some embodiments, the dose is about 20 mg BID. In other embodiments, the dose is about 30 mg BID. In other embodiments, the dose is about 40 mg BID.

In some embodiments, stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt, or solvate thereof is administered orally in a dosage form such as a tablet and a capsule.

In certain embodiments, apremilast is orally administered to a patient having obesity at a starting dose of about 10 mg, 20 mg, 30 mg, or 40 mg twice daily. In some embodiments, the maximum daily dose is about 20 mg to about 40 mg. In some embodiments, the maximum daily dose is about 30 mg to about 60 mg. In some embodiments, the maximum daily dose is about 40 mg to about 100 mg.

In some embodiments, the stereomerically pure (+)-2-[1-(3-ethoxy-4-methoxyphenyl)-2-methylsulfonylethyl]-4-acetylaminoisoindoline-1,3-dione comprises greater than about 90% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione comprises greater than about 95% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione comprises greater than about 96% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione comprises greater than about 97% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione comprises greater than about 98% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione comprises greater than about 99% by weight of (+) isomer based on the total weight percent of the compound.

In some embodiments, the stereomerically pure (+)-2-[1-(3-ethoxy-4-methoxyphenyl)-2-methylsulfonylethyl]-4-acetylaminoisoindoline-1,3-dione is administered in an amount of about 20 mg twice a day following the initial titration schedule.

In some embodiments, the stereomerically pure (+)-2-[1-(3-ethoxy-4-methoxyphenyl)-2-methylsulfonylethyl]-4-acetylaminoisoindoline-1,3-dione is administered in an amount of about 30 mg twice a day following the initial titration schedule.

In some embodiments, the stereomerically pure (+)-2-[1-(3-ethoxy-4-methoxyphenyl)-2-methylsulfonylethyl]-4-acetylaminoisoindoline-1,3-dione is administered in an amount of about 40 mg twice a day following the initial titration schedule.

In some embodiments, the stereomerically pure (+)-2-[1-(3-ethoxy-4-methoxyphenyl)-2-methylsulfonylethyl]-4-acetylaminoisoindoline-1,3-dione is administered once or twice daily.

In some embodiments, the stereomerically pure (+)-2-[1-(3-ethoxy-4-methoxyphenyl)-2-methylsulfonylethyl]-4-acetylaminoisoindoline-1,3-dione is administered in tablet form. In some embodiments, the tablet comprises a 10 mg, 20 mg, 30 mg, or 40 mg dose of stereomerically pure (+)-2-[1-(3-ethoxy-4-methoxyphenyl)-2-methylsulfonylethyl]-4-acetylaminoisoindoline-1,3-dione.

In some embodiments, the tablet further comprises lactose monohydrate, microcrystalline cellulose, croscarmellose sodium, magnesium stearate, polyvinyl alcohol, titanium dioxide, polyethylene glycol, and talc. In some embodiments, the tablet further comprises iron oxide red. In some embodiments, the tablet further comprises iron oxide yellow. In some embodiments, the tablet further comprises iron oxide black.

In one embodiment, the stereomerically pure (+)-2-[1-(3-ethoxy-4-methoxyphenyl)-2-methylsulfonylethyl]-4-acetylaminoisoindoline-1,3-dione is administered in tablet form comprising a 10 mg dose of stereomerically pure (+)-2-[1-(3-ethoxy-4-methoxyphenyl)-2-methylsulfonylethyl]-4-acetylaminoisoindoline-1,3-dione, lactose monohydrate, microcrystalline cellulose, croscarmellose sodium, magnesium stearate, polyvinyl alcohol, titanium dioxide, polyethylene glycol, talc, and iron oxide red.

In one embodiment, the stereomerically pure (+)-2-[1-(3-ethoxy-4-methoxyphenyl)-2-methylsulfonylethyl]-4-acetylaminoisoindoline-1,3-dione is administered in tablet form comprising a 20 mg dose of stereomerically pure (+)-2-[1-(3-ethoxy-4-methoxyphenyl)-2-methylsulfonylethyl]-4-acetylaminoisoindoline-1,3-dione, lactose monohydrate, microcrystalline cellulose, croscarmellose sodium, magnesium stearate, polyvinyl alcohol, titanium dioxide, polyethylene glycol, talc, iron oxide red, and iron oxide yellow.

In one embodiment, the stereomerically pure (+)-2-[1-(3-ethoxy-4-methoxyphenyl)-2-methylsulfonylethyl]-4-acetylaminoisoindoline-1,3-dione is administered in tablet form comprising a 30 mg dose of stereomerically pure (+)-2-[1-(3-ethoxy-4-methoxyphenyl)-2-methylsulfonylethyl]-4-acetylaminoisoindoline-1,3-dione, lactose monohydrate, microcrystalline cellulose, croscarmellose sodium, magnesium stearate, polyvinyl alcohol, titanium dioxide, polyethylene glycol, talc, and iron oxide red, iron oxide yellow, and iron oxide black.

In some embodiments, the invention provided herein, further comprise administering to the patient a therapeutically effective amount of a second active agent. In some embodiments, the invention further comprises the administration of a therapeutically effective amount of medications available for the management of obesity, including but not limited to, gastrointestinal agents (*e.g*., orlistat), CNS stimulants and anorexiants (*e.g*., lorcaserin, topiramate, phentermine, diethylpropion, phendimetrazine, and benzphetamine).

In some embodiments, the invention further comprises the administration of a therapeutically or prophylactically effective amount of one or more second active agents, including but not limited to, an analgesic, an anti-inflammatory agent, a COX-2 inhibitor, an opioid, a corticosteroid, a biologic agent, and an immunosuppressant. In some embodiments, the second active agent is a non-steroidal anti-inflammatory drug (*i.e*., NSAID such as celecoxib, diclofenac, ibuprofen, indomethacin, meloxicam, naproxen, and piroxicam). In some embodiments, the second active agent is a disease-modifying antirheumatic drug (*i.e*., DMARD such as methotrexate, leflunomide, sulfasalazine and hydroxychloroquine).

In some embodiments, the invention further comprises other weight reduction measures, such as change in dietary habits, exercise programs, or psychological support programs.

In some embodiments, the patient has received prior treatment for obesity or arthritis. In some embodiments, the patient is relapsed or refractory to prior treatment.

In some embodiments, the invention comprises administering stereomerically pure (+)-2-[1-(3-ethoxy-4-methoxyphenyl)-2-methylsulfonylethyl]-4-acetylaminoisoindoline-1,3-dione, substantially free of any salt or solvate forms of (+)-2-[1-(3-ethoxy-4-methoxyphenyl)-2-methylsulfonylethyl]-4-acetylaminoisoindoline-1,3-dione.

In some embodiments, the invention comprises administering a pharmaceutically acceptable salt of stereomerically pure (+)-2-[1-(3-ethoxy-4-methoxyphenyl)-2-methylsulfonylethyl]-4-acetylaminoisoindoline-1,3 -dione.

In some embodiments, the invention comprises administering a pharmaceutically acceptable solvate of stereomerically pure (+)-2-[1-(3-ethoxy-4-methoxyphenyl)-2-methylsulfonylethyl]-4-acetylaminoisoindoline-1,3 -dione.

### 5.2.1 Combination Therapy

In particular embodiments, apremilast is administered in combination with another drug ("second active agent") for treating, managing and/or preventing obesity.

In certain embodiments, the invention encompasses synergistic combinations for the treatment, prevention and/or management of obesity. Apremilast may also be used to alleviate adverse effects associated with some second active agents.

One or more second active agents can be used in the invention together with apremilast. Second active agents can be administered before, after or simultaneously with premilast. In some embodiments, the invention comprises the administration of a therapeutically effective amount of medications for treating obesity, including but not limited to, gastrointestinal agents (*e.g*., orlistat), CNS stimulants and anorexiants (*e.g*., lorcaserin, phentermine, diethylpropion, phendimetrazine, and benzphetamine).

In one embodiment, the second active agent is selected from the group consisting of an anti-inflammatory agent, an immunosuppressant, mycophenolate mofetil, a biologic agent, or a Cox-2 inhibitor.

In some embodiments, the second active agents may include, but are not limited to, anti-inflammatories such as NSAIDs including, but not limited to, diclofenac (*e.g*., ARTHROTEC®), diflunisal (*e.g*., DOLOBID®), etodolac (*e.g*., LODINE®), fenoprofen (*e.g*., NALFON®), ibuprofen (*e.g*., ADVIL, CHILDREN'S ADVIL/MOTRIN, MEDIPREN, MOTRIN, NUPRIN or PEDIACARE FEVER ®), indomethacin (*e.g*., ARTHREXIN®), ketoprofen (*e.g*., ORUVAIL®), ketorolac (*e.g*., TORADOL®), fosfomycin tromethamine (*e.g*., MONURAL®), meclofenamate (*e.g*., Meclomen®), nabumetone (*e.g*., RELAFEN®), naproxen (*e.g*., ANAPROX®, ANAPROX® DS, EC-NAPROSYN®, NAPRELAN® or NAPROSYN®), oxaprozin (*e.g*., DAYPRO®), piroxicam (*e.g*., FELDENE®), sulindac (*e.g*., CLINORIL®), and tolmetin (*e.g*., TOLECTIN® DS or TOLECTIN®).

In other embodiments, the second active agents may include, but are not limited to, disease-modifying antirheumatic drugs (DMARDs) or immnunosuppressants such as, but not limited to, methotrexate (Rheumatrex®), sulfasalazine (Azulfidine®), leflunomide (Arava®), and cyclosporine (Sandimmune® or Neroal®).

In other embodiments, the second active agent is an oral corticosteroid, such as, but not limited to, budesonide (Entocort®), dexamethazone, fludrocortisone (Florinef®, Florinef® acetate), hydrocortisone, methylprednisone, prednisolone, and prednisone.

In other embodiments, second active agents may include, but are not limited to, mycophenolate mofetil (CellCept®), an immunosuppressive agent widely used in organ transplantation and gaining favor in treating autoimmune and inflammatory skin disorders.

In further embodiments, second active agents may include, but are not limited to, biologic agents such as etanercept (Enbrel®), infliximab (Remicade®) and adalimumab (Humira®).

In further embodiments, second active agents may include, but are not limited to, Cox-2 inhibitors such as celecoxib (Celebrex®), valdecoxib (Bextra®) and meloxicam (Mobic®).

In some embodiments, one or more selective active agents are selected from the group consisting of acitretin, adalimumab, alclometasone, alefacept, aloe vera, amcinonide, ammonium lactate/urea, ammonium lactate/halobetasol, anthralin, benzocaine/pyrilamine/zinc oxide, betamethasone, betamethasone/calcipotriene, calcipotriene, clobetasol, clocortolone, coal tar, coal tar/salicylic acid, corticotropin, cyclosporine, desonide, desoximetasone, diflorasone, fluocinonide, flurandrenolide, halcinonide, halobetasol, hydrocortisone, hydrocortisone/pramoxine, hydroxyurea, infliximab, methotrexate, methoxsalen, mometasone, pramoxine, prednisone, prednisolone, prednicarbate, resorcinol, tazarotene, triamcinolone and ustekinumab.

In some embodiments, one or more selective active agents are selected from the group consisting of abatacept, acetaminophen, acetaminophen/hydrocodone, acetaminophen/tramadol, adalimumab, alemtuzumab, aluminum hydroxide/aspirin/calcium carbonate/magnesium hydroxide, anakinra, aspirin, auranofin, aurothioglucose, atorvastatin, azathioprine, celecoxib, certolizumab, chondroitin, cortisone, corticotropin, cyclophosphamide, cyclosporine, daclizumab, dexamethasone, diclofenac, diclofenac/misoprostol, diflunisal, doxycycline, esomeprazole, esomeprazole/naproxen, etanercept, etodolac, famotidine, famotidine/ibuprofen, fenoprofen, flurbiprofen, glucosamine, gold sodium thiomalate, golimumab, hydroxychloroquine, ibuprofen, indomethacin, infliximab, interferon, interferon gamma-lb, ketoprofen, lansoprazole, lansoprazole/naproxen, leflunomide, levamisole, meclofenamate, meloxicam, methotrexate, methylprednisone, methylprednisolone, methyl salicylate, minocycline, mycophenolate mofetil, nabumetone, naproxen, oxaprozin, penicillamine, phenytoin, piroxicam, prednisone, primrose oil, rituximab, rofecoxib, salsalate, sulindac, sulfasalazine, tetracycline, tocilizumab, tofacitinib, tolmetin, tramadol, triamcinolone, trolamine salicylate and valdecoxib.

In some embodiments, one or more selective active agents are selected from the group consisting of abatacept, acetaminophen, acetaminophen/hydrocodone, acetaminophen/tramadol, acitretin, adalimumab, alclometasone, alefacept, alemtuzumab, aloe vera, aluminum hydroxide/aspirin/calcium carbonate/magnesium hydroxide, amcinonide, ammonium lactate/urea, ammonium lactate/halobetasol, anakinra, anthralin, aspirin, auranofin, aurothioglucose, atorvastatin, azathioprine, benzocaine/pyrilamine/zinc oxide, betamethasone, betamethasone/calcipotriene, calcipotriene, celecoxib, certolizumab, chondroitin, clobetasol, clocortolone, coal tar, coal tar/salicylic acid, corticotropin, cortisone, cyclophosphamide, cyclosporine, daclizumab, desonide, desoximetasone, dexamethasone, diclofenac, diclofenac/misoprostol, diflorasone, diflunisal, doxycycline, esomeprazole, esomeprazole/naproxen, etanercept, etodolac, famotidine, famotidine/ibuprofen, fenoprofen, fluocinonide, flurandrenolide, flurbiprofen, fostamatinib, glucosamine, gold sodium thiomalate, golimumab, halcinonide, halobetasol, hydrocortisone, hydrocortisone/pramoxine, hydroxyurea, hydroxychloroquine, ibuprofen, indomethacin, infliximab, interferon, interferon gamma-lb, ibrutinib, ketoprofen, lansoprazole, lansoprazole/naproxen, leflunomide, lenalidomide, levamisole, meclofenamate, meloxicam, methotrexate, methoxsalen, methylprednisone, methylprednisolone, methyl salicylate, minocycline, mometasone, mycophenolate mofetil, nabumetone, naproxen, oxaprozin, penicillamine, phenytoin, piroxicam, pomalidomide, pramoxine, prednisone, prednisolone, prednicarbate, primrose oil, resorcinol, rituximab, rofecoxib, salsalate, sulindac, sulfasalazine, tazarotene, tetracycline, tocilizumab, tofacitinib, tolmetin, tramadol, triamcinolone, trolamine salicylate, ustekinumab, valdecoxib, 3-(5-amino-2-methyl-4-oxo-4H-quinazolin-3-yl)-piperidine-2,6-dione, and (S)-3-(4-((4-(morpholinomethyl)benzyl)oxy)-1-oxoisoindolin-2-yl)piperidine-2,6-dione.

In some embodiments, one or more selective active agents are selected from the group consisting of a Btk inhibitor, a cereblon targeting agent, a Tyk2 inhibitor, a Syk inhibitor, a JNK inhibitor, a MK2 inhibitor, a ERP5 inhibitor, a PD-1 inhibitor, a TIMP-3 inhibitor, a IKK-2 inhibitor, a LH2B inhibitor, a PKC-theta inhibitor, a IRAK4 inhibitor, a ROCK inhibitor, and a ROR-gamma-T inhibitor.

Administration of apremilast and a second active agent to a patient can occur simultaneously or sequentially by the same or different routes of administration. The suitability of a particular route of administration employed for a particular second active agent will depend on the second active agent itself (*e.g*., whether it can be administered orally or topically without decomposition prior to entering the blood stream) and the subject being treated. Particular routes of administration for the second active agents or ingredients are known to those of ordinary skill in the art. *See, e.g.,* The Merck Manual, 448 (17th ed., 1999).

The amount of a second active agent administered can be determined based on the specific agent used, the subject being treated, the severity and stage of disease and the amount(s) of apremilast and any optional additional second active agents concurrently administered to the patient. Those of ordinary skill in the art can determine the specific amounts according to conventional procedures known in the art. In the beginning, one can start from the amount of the second active agent that is conventionally used in the therapies and adjust the amount according to the factors described above. *See, e.g.,* Physician's Desk Reference (59th Ed., 2005).

In certain embodiments, the second active agent is administered orally, topically, intravenously or subcutaneously and once to four times daily in an amount of from about 1 to about 1,000 mg, from about 5 to about 500 mg, from about 10 to about 350 mg or from about 50 to about 200 mg. The specific amount of the second active agent will depend on the specific agent used, the age of the subject being treated, the severity and stage of disease and the amount(s) of apremilast and any optional additional second active agents concurrently administered to the patient.

### 5.3 Apremilast

Without being limited by theory, apremilast is believed to be (+) enantiomer of 2-[1-(3-ethoxy-4-methoxyphenyl)-2-methanesulfonylethyl]-4-acetylaminoisoindolin-1,3-dione having the following structure:

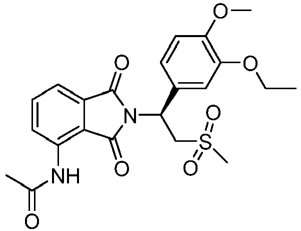

Apremilast may be prepared according to methods disclosed in U.S. Patent Nos. 6,962,940; 7,208,516; 7,427,638; or 7,893,101. In a specific method, apremilast may be prepared, for example, by the following process.

A stirred solution of 1-(3-ethoxy-4-methoxyphenyl)-2-methylsulfonylethylamine (1.0 g, 3.7 mmol) and 3-acetamidophthalic anhydride (751 mg, 3.66 mmol) in acetic acid (20 mL) was heated at reflux for 15 h. The solvent was removed *in vacuo* to yield an oil. Chromatography of the resulting oil yielded the product as a yellow solid (1.0 g, 59% yield): mp, 144 °C; ¹H NMR (CDCl₃) δ: 1.47 (t, J=7.0 Hz, 3H, CH₃), 2.26 (s, 3H, CH₃), 2.88 (s, 3H, CH₃), 3.75 (dd, J=4.4, 14.3 Hz, 1H, CH), 3.85 (s, 3H, CH3), 4.11 (q, J=7 Hz, 2H, CH₂), 5.87 (dd, J=4.3, 10.5 Hz, 1H, NCH), 6.82-6.86 (m, 1H, Ar), 7.09-7.11 (m, 2H, Ar), 7.47 (d, J= 7 Hz, 1H, Ar), 7.64 (t, J= 8 Hz, 1H, Ar), 8.74 (d, J= 8 Hz, 1H, Ar), 9.49 (br s, 1H, NH); ¹³C NMR (CDCl₃) δ: 14.61, 24.85, 41.54, 48.44, 54.34, 55.85, 64.43, 111.37, 112.34, 115.04, 118.11, 120.21, 124.85, 129.17, 130.96, 136.01, 137.52, 148.54, 149.65, 167.38, 169.09, 169.40; Anal Calc'd. for C₂₂H₂₄NO₇S: C, 57.38; H, 5.25; N, 6.08. Found: C, 57.31; H, 5.34; N, 5.83.

Preparation of 3-aminophthalic acid: 10% Pd/C (2.5 g), 3-nitrophthalic acid (75.0 g, 355 mmol) and ethanol (1.5 L) were charged to a 2.5 L Parr hydrogenator under a nitrogen atmosphere. Hydrogen was charged to the reaction vessel for up to 55 psi. The mixture was shaken for 13 hours, maintaining hydrogen pressure between 50 and 55 psi. Hydrogen was released and the mixture was purged with nitrogen 3 times. The suspension was filtered through a celite bed and rinsed with methanol. The filtrate was concentrated *in vacuo.* The resulting solid was reslurried in ether and isolated by vacuum filtration. The solid was dried *in vacuo* to a constant weight, affording 54 g (84% yield) of 3-aminopthalic acid as a yellow product. ¹H-NMR (DMSO-d6) δ: 3.17 (s, 2H), 6.67 (d, 1H), 6.82 (d, 1H), 7.17 (t, 1H), 8-10 (br, s, 2H); ¹³C-NMR (DMSO-d6) δ: 112.00, 115.32, 118.20, 131.28, 135.86, 148.82, 169.15, 170.09.

Preparation of 3-aminophthalic anhydride: A 1 L 3-necked round bottom flask was equipped with a mechanical stirrer, thermometer, and condenser and charged with 3-aminophthalic acid (108 g, 596 mmol) and acetic anhydride (550 mL). The reaction mixture was heated to reflux for 3 hours and cooled to about 25 °C and further to 0-5 °C for another 1 hour. The crystalline solid was collected by vacuum filtration and washed with ether. The solid product was dried *in vacuo* at ambient temperature to a constant weight, giving 75 g (61% yield) of 3-acetamidopthalic anhydride as a white product. ¹H-NMR (CDCl₃) δ: 2.21 (s, 3H), 7.76 (d, 1H), 7.94 (t, 1H), 8.42 (d, 1H), 9.84 (s, 1H).

Resolution of 2-(3-ethoxy-4-methoxyphenyl-1-(methylsulphonyl)-eth-2-ylamine: A 3 L 3-necked round bottom flask was equipped with a mechanical stirrer, thermometer, and condenser and charged with 2-(3-ethoxy-4-methoxyphenyl)-1-(methylsulphonyl)-eth-2-ylamine (137.0 g, 500 mmol), N-acetyl-L-leucine (52 g, 300 mmol), and methanol (1.0 L). The stirred slurry was heated to reflux for 1 hour. The stirred mixture was allowed to cool to ambient temperature and stirring was continued for another 3 hours at ambient temperature. The slurry was filtered and washed with methanol (250 L). The solid was air-dried and then dried *in vacuo* at ambient temperature to a constant weight, giving 109.5 g (98% yield) of the crude product (85.8% ee). The crude solid (55.0 g) and methanol (440 mL) were brought to reflux for 1 hour, cooled to room temperature and stirred for an additional 3 hours at ambient temperature. The slurry was filtered and the filter cake was washed with methanol (200 mL). The solid was air-dried and then dried *in vacuo* at 30 °C to a constant weight, yielding 49.6 g (90% recovery) of (*S*)-2-(3-ethoxy-4-methoxyphenyl)-1-(methylsulphonyl)-eth-2-ylamine-N-acetyl-L-leucine salt (98.4% ee). Chiral HPLC (1/99 EtOH/20 mM KH₂PO₄ @ pH 7.0, Ultron Chiral ES-OVS from Agilent Technologies, 150 mm x 4.6 mm, 0.5 mL/min., @ 240 nm): 18.4 min (*S*-isomer, 99.2%), 25.5 min (*R*-isomer, 0.8%).

Final preparation of apremilast: A 500 mL 3-necked round bottom flask was equipped with a mechanical stirrer, thermometer, and condenser. The reaction vessel was charged with (*S*)-2-(3-ethoxy-4-methoxyphenyl)-1-(methylsulphonyl)-eth-2-yl amine N-acetyl-L-leucine salt (25 g, 56 mmol, 98% ee), 3-acetamidophthalic anhydride (12.1 g, 58.8 mmol), and glacial acetic acid (250 mL). The mixture was refluxed over night and then cooled to <50° C. The solvent was removed *in vacuo,* and the residue was dissolved in ethyl acetate. The resulting solution was washed with water (250 mL x 2), saturated aqueous NaHCO₃ (250 mL x 2), brine (250 mL x 2), and dried over sodium sulphate. The solvent was evaporated *in vacuo,* and the residue recrystallized from a binary solvent containing ethanol (150 mL) and acetone (75 mL). The solid was isolated by vacuum filtration and washed with ethanol (100 mL x 2). The product was dried *in vacuo* at 60 °C to a constant weight, affording 19.4 g (75% yield) of apremilast with 98% ee. Chiral HPLC (15/85 EtOH/20 mM KH₂PO₄ @ pH 5, Ultron Chiral ES-OVS from Agilent Technology, 150 mm x 4.6 mm, 0.4 mL/min, @ 240 nm): 25.4 min (S-isomer, 98.7%), 29.5 min (*R*-isomer, 1.2%). ¹H-NMR (CDCl₃) δ: 1.47 (t, 3H), 2.26 (s, 3H), 2.87 (s, 3H), 3.68-3.75 (dd, 1H), 3.85 (s, 3H), 4.07-4.15 (q, 2H), 4.51-4.61 (dd, 1H), 5.84-5.90 (dd, 1H), 6.82-8.77 (m, 6H), 9.46 (s, 1H); ¹³C-NMR (DMSO-d6) δ: 14.66, 24.92, 41.61, 48.53, 54.46, 55.91, 64.51, 111.44, 112.40, 115.10, 118.20, 120.28, 124.94, 129.22, 131.02, 136.09, 137.60, 148.62, 149.74, 167.46, 169.14, 169.48.

### 5.4 Pharmaceutical Compositions and Dosage Forms

Pharmaceutical compositions can be used in the preparation of individual, single unit dosage forms. Pharmaceutical compositions and dosage forms can comprise apremilast or a pharmaceutically acceptable salt or solvate thereof and a second active agent. Examples of the optional second active agents are disclosed herein (*see, e.g.,* section 4.2.1). Pharmaceutical compositions and dosage forms can further comprise one or more carriers, excipients or diluents.

The pharmaceutical compositions provided herein are suitable for oral administration can be presented as discrete dosage forms, such as, but not limited to, tablets (*e.g*., chewable tablets), caplets, capsules and liquids (*e.g*., flavored syrups). Such dosage forms contain predetermined amounts of active ingredients and can be prepared by methods of pharmacy well known to those skilled in the art. *See generally,* Remington's Pharmaceutical Sciences, 20th ed., Mack Publishing, Easton PA (2000).

Typical oral dosage forms are prepared by combining the active ingredients in an intimate admixture with at least one excipient according to conventional pharmaceutical compounding techniques. Excipients can take a wide variety of forms depending on the form of preparation desired for administration. Non-limiting examples of excipients suitable for use in oral liquid or aerosol dosage forms include water, glycols, oils, alcohols, flavoring agents, preservatives and coloring agents. Non-limiting examples of excipients suitable for use in solid oral dosage forms (*e.g*., powders, tablets, capsules and caplets) include starches, sugars, microcrystalline cellulose, diluents, granulating agents, lubricants, binders and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers or both and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Non-limiting examples of excipients that can be used in oral dosage forms include binders, fillers, disintegrants and lubricants. Non-limiting examples of binders suitable for use in pharmaceutical compositions and dosage forms include corn starch, potato starch or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g*., Nos. 2208, 2906, 2910), microcrystalline cellulose and mixtures thereof.

Non-limiting examples of suitable forms of microcrystalline cellulose include, but are not limited to, the materials sold as AVICEL® (microcrystalline cellulose) PH-101, AVICEL® (microcrystalline cellulose) PH-103, AVICEL RC-581® (crystalline cellulose and carboxymethylcellulose sodium), AVICEL® (microcrystalline cellulose) PH-105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC-581® (crystalline cellulose and carboxymethylcellulose sodium). Suitable anhydrous or low moisture excipients or additives include AVICEL-PH-103™® (microcrystalline cellulose) PH-103 and Starch 1500® LM (pregelatinized starch).

Non-limiting examples of fillers suitable for use in the pharmaceutical compositions and dosage forms disclosed herein include talc, calcium carbonate (*e.g*., granules or powder), microcrystalline cellulose, powdered cellulose, dextrates, kaolin, mannitol, silicic acid, sorbitol, starch, pre-gelatinized starch and mixtures thereof. The binder or filler in pharmaceutical compositions is typically present in from about 50 to about 99 weight percent of the pharmaceutical composition or dosage form.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms. The amount of disintegrant used varies based upon the type of formulation and is readily discernible to those of ordinary skill in the art. Typical pharmaceutical compositions comprise from about 0.5 to about 15 weight percent of disintegrant, preferably from about 1 to about 5 weight percent of disintegrant.

Non-limiting examples of disintegrants that can be used in pharmaceutical compositions and dosage forms include agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums and mixtures thereof.

Non-limiting examples of lubricants that can be used in pharmaceutical compositions and dosage forms include calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil and soybean oil), zinc stearate, ethyl oleate, ethyl laureate, agar and mixtures thereof. Additional lubricants include, for example, a syloid silica gel (AEROSIL200® (silica), manufactured by W.R. Grace Co. of Baltimore, MD), a coagulated aerosol of synthetic silica (marketed by Degussa Co. of Plano, TX), CAB-O-SIL® (fumed silica) (a pyrogenic silicon dioxide product sold by Cabot Co. of Boston, MA) and mixtures thereof. If used at all, lubricants are typically used in an amount of less than about 1 weight percent of the pharmaceutical compositions or dosage forms into which they are incorporated.

Non-limiting examples of dosage forms include tablets; caplets; capsules, such as soft elastic gelatin capsules; sachets; troches; lozenges; dispersions; suppositories; powders; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions or water-in-oil emulsions), solutions and elixirs.

The composition, shape and type of dosage forms will typically vary depending on their use. For example, a dosage form used in the acute treatment of a disease may contain larger amounts of one or more of the active ingredients it comprises than a dosage form used in the chronic treatment of the same disease. These and other ways in which specific dosage forms will vary from one another will be readily apparent to those skilled in the art. *See, e.g.,* Remington's Pharmaceutical Sciences, 20th ed., Mack Publishing, Easton PA (2,000).

Typical pharmaceutical compositions and dosage forms comprise one or more excipients. Suitable excipients are well known to those skilled in the art of pharmacy and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. The suitability of a particular excipient may also depend on the specific active ingredients in the dosage form. For example, the decomposition of some active ingredients can be accelerated by some excipients such as lactose or when exposed to water. Active ingredients that comprise primary or secondary amines are particularly susceptible to such accelerated decomposition.

In certain embodiments, provided herein are anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Non-limiting examples of suitable packaging include hermetically sealed foils, plastics, unit dose containers (*e.g*., vials), blister packs and strip packs.

Also provided herein are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers or salt buffers. Like the amounts and types of excipients, the amounts and specific types of active ingredients in a dosage form may differ depending on factors such as, but not limited to, the route by which it is to be administered to patients. However, typical oral dosage forms comprise apremilast in an amount of 10 mg, 20 mg, 25 mg, 30 mg, 35 mg, or 40 mg. In a particular embodiments, the oral dosage forms are 10 mg, 20 mg, 25mg, 30 mg, 35 mg, or 40 mg tablets.

### 5.5 Delayed Release Dosage Forms

In certain embodiments, active ingredients can be administered by controlled release means or by delivery devices that are well known to those of ordinary skill in the art. Non-limiting examples of controlled release means or delivery devices include those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556 and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres or a combination thereof to provide the desired release profile in varying proportions. Suitable controlled-release formulations known to those of ordinary skill in the art, including those described herein, can be readily selected for use with the active ingredients. In certain embodiments, provided herein are single unit dosage forms suitable for oral administration such as, but not limited to, tablets, capsules, gelcaps and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency and increased patient compliance. In addition, controlled-release formulations can be used to affect the time of onset of action or other characteristics, such as blood levels of the drug and can thus affect the occurrence of side (*e.g*., adverse) effects.

Most controlled-release formulations are designed to initially release an amount of drug (active ingredient) that promptly produces the desired therapeutic effect and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water or other physiological conditions or compounds.

### 6. EXAMPLES

Some embodiments are illustrated by the following non-limiting examples. The examples should not be construed as a limitation in the scope thereof.

### 6.1 Biological Activity of Apremilast in Preclinical Models

Arthritic conditions are considered to be a Th1 autoimmune disease because of the involvement of pro-inflammatory cytokines, interferon (INF)γ and tumor necrosis factor (TNF)-α. Elevation of cyclic nucleotide adenosine 3',5'-cyclic monophosphate (cAMP) inhibits the release of inflammatory mediators, including TNF-α. A cellular mechanism for the inactivation of cAMP is its breakdown by cyclic nucleotide phosphodiesterases (PDEs). The inhibition of PDE4 is particularly effective in the inhibition of inflammatory mediator release. Thus, compounds that inhibit PDE4 specifically may inhibit inflammation with a minimum of unwanted side effects.

**Inhibition of TNF-α:** Apremilast inhibits TNF-α production in PBMCs (IC₅₀ of 77 nM), in human whole blood (IC₅₀ of 294), and in a mouse model (EC₅₀ of 0.05 mg/kg). The test methods were as described in WO 03/080049; Muller et al., J. Med. Chem., 1996, 39:3238; and Muller et al., Bioorg. Med. Chem. Lett., 1999, 9:1625-30.

**Inhibition of PDE4:** Phosphodiesterase 4 enzyme was purified from U937 human monocytic cells by gel filtration chromatography, and phosphodiesterase reactions were carried out as previously described. *See* Muller et al., Bioorg. Med. Chem. Lett., 1998, 8(19): 2669-2674. Briefly, reactions were carried out in 96-well deep-well plates in 50 mM Tris HCl pH 7.5, 5 mM MgC12, 1 µM cyclic adenosine monophosphate (cAMP), plus 10 nM [3H]-cAMP for 45 min at 30°C. The reactions were terminated by boiling, treated with 1 mg/ml snake venom, and separated using AG-1X8 ion exchange resin (BioRad). Reactions consumed less than 15% of available substrate. Apremilast inhibited PDE4 with an IC₅₀ of 73.5 nM.

**PDE4 selectivity:** Apremilast selectively inhibits PDE4 over PDE1 (23% inhibition at 10 µM), PDE2 (6% inhibition at 10 µM), PDE3 (20% inhibition at 10 µM), PDE5 (3% inhibition at 10 µM), PDE6 (-6% inhibition at 10 µM) and PDE7 (IC₅₀ of 20.5 µM). PDE1, 2, 3 and 5 enzyme assays were prepared as described by Hidaka and Asano. Biochem. Biophys. Acta., 1976, 429:485; *see also* Nicholsen et al., Trends Pharmaco. Sci., 1991, 12:19. The PDE6 enzyme assay was prepared according to Baehr et al., J. Biol. Chem., 1979, 254:11669 and Gillespie et al., Mol. Pharm., 1989, 36: 773. The PDE7 enzyme assay was prepared according to Bloom and Beavo. Proc. Natl. Acad. Sci. USA, 1996, 93:14188-92.

### 6.2 Clinical Data of Weight Loss using Apremilast

Apremilast has shown benefit of weight loss in both Phase 2 and Phase 3 Psoriatic Arthritis (PSA) and Psoriasis (PSOR) trials. Unlike weight decrease observed as a side effect in COPD patients treated with other PDE4 inhibitor, weight loss was observed in treatment with apremilast for patients with a mean BMI of 30 or more, which is the clinical definition of obesity. Weight loss was observed in the population of over 4,000 patients treated with apremilast in the Phase 2 and Phase 3 psoriatic arthritis and psoriasis clinical trial programs.

### Psoriatic Arthritis (PsA) Phase 3 Data Pool

In the psoriatic arthritis Phase 3 study, weight decrease was reported by 2 (0.3%) subjects who received placebo and 25 (1.3%) subjects who received apremilast, including 10 (1.0%) subjects in apremilast 20 mg (hereinafter "APR 20") BID group and 15 (1.5%) subjects in the apremilast 30mg (hereinafter "APR 30") BID group.

### Psoriasis (PSOR) Phase 3 Data Pool

In the psoriasis Phase 3 study, weight decrease was reported by 1 (0.2%) subjects who received placebo and 18 (1.5%) subjects who received APR 30mg BID.

### Apremilast Data Pool

Similar results of weight loss were reported for the Apremilast Data Pool. A weight decrease was reported during the Placebo-controlled Period (24 weeks) in 3 (0.2%) subjects who received placebo and 26 (0.9%) subjects who received apremilast, including 8 (0.8%) subject in the APR 20 BID group, and 18 (1.1%) subjects in the APR 30 BID group.

In the Apremilast-exposure Period, weight decrease was reported in 17 (1.2%) subject in the APR 20 BID group and 36 (1.5%) subjects in the APR 30 BID group.

### Detailed Analysis of Weight Change

### Psoriatic Arthritis (PsA) Phase 3 Data Pool

For the subjects as initially treated at Week 0 population in the PsA Phase 3 Data Pool, the placebo group had a mean weight change from baseline of 0.13 kg compared with a mean weight change from baseline of -1.05 kg in the APR 20 BID group and -0.97 kg in the APR 30 BID group at the end of the Placebo-controlled Period or Week 24. At the end of the Apremilast-exposure Period, the APR 20 BID and APR 30 BID groups had a mean weight change from baseline of -1.13 kg and -1.26 kg, respectively (Table 1).

**Table 1: PsA Phase 3 Data Pool: Change in Weight From Baseline at End of Period (Subjects as Initially Treated at Week 0)**

| **Weight, kg** | | | | **Change From Baseline (kg)** | |
|---|---|---|---|---|---|
| **Time Point Treatment Group** | **n^{a}** | **Baseline Mean ± SD** | **End of Period Mean ± SD** | **Mean ± SD** | **Median (Min, Max)** |
| End of Period (PBC)^{b} | | | | | |
| Placebo | 665 | 85.28 ± 20.400 | 85.41 ± 20.347 | 0.13 ± 2.510 | 0.00 (-14.6, 13.0) |
| APR 20 BID | 669 | 85.51 ± 21.327 | 84.47 ± 20.734 | -1.05 ± 3.366 | -0.50 (-17.2, 11.5) |
| APR 30 BID | 663 | 84.87 ± 19.811 | 83.90 ± 19.670 | -0.97 ± 3.016 | -0.60 (-13.7, 11.4) |

| End of Period (APR)^{c} | | | | | |
|---|---|---|---|---|---|
| APR 20 BID | 669 | 85.51 ± 21.327 | 84.38 ± 20.636 | -1.13 ± 4.491 | -0.60 (-44.3, 14.0) |
| APR 30 BID | 663 | 84.87 ± 19.811 | 83.61 ± 19.766 | -1.26 ± 4.120 | -1.00 (-23.0, 17.1) |

| | | | | | |
|---|---|---|---|---|---|
| APR 20/30 BID = apremilast 20/30 mg twice daily; max = maximum; min = minimum; PBC = placebo-controlled; PsA = psoriatic arthritis. a At the end of period, n = number of subjects with a baseline value and at least 1 postbaseline value. b The last postbaseline value during the Placebo-controlled Period, including data up to 28 days after the last dose of investigational product but excluding the observational follow-up visit. c The last postbaseline value during apremilast exposure, including data up to 28 days after the last dose of apremilast but excluding the observational follow-up visit. Note: Data collected later than 28 days after the last dose of investigational product are excluded. Data in the randomized withdrawal phase (Weeks 32 to 52) from 28 days after initiating placebo until apremilast was resumed are excluded. | | | | | |

For the Apremilast subjects as treated population, mean weight change from baseline for the APR 20 BID group was -1.25 kg at Week 52 and -1.70 kg at Week 78. For the APR 30 BID group, mean weight change from baseline was -1.68 kg at Week 52 and -2.14 kg at Week 78 (Table 2).

**Table 2: PsA Phase 3 Data Pool: Change in Weight From Baseline by Time Point (Apremilast Subjects as Treated)**

| **Weight, kg** | | | | **Change From Baseline (kg)** | |
|---|---|---|---|---|---|
| **Time Point Treatment Group** | **n^{a}** | **Baseline Mean ± SD** | **Time Point Mean ± SD** | **Mean ± SD** | **Median (Min, Max)** |
| Week 52 | | | | | |
| APR 20 BID | 446 | 86.13 ± 20.979 | 84.89 ± 20.190 | -1.25 ± 4.223 | -1.00 (-26.0, 12.5) |
| APR 30 BID | 467 | 85.84 ± 19.478 | 84.16 ± 19.253 | -1.68 ± 4.049 | -1.30 (-23.0, 11.8) |

| Week 78 | | | | | |
|---|---|---|---|---|---|
| APR 20 BID | 165 | 88.33 ± 21.870 | 86.63 ± 19.721 | -1.70 ± 6.192 | -1.00 (-44.3, 14.0) |
| APR 30 BID | 173 | 85.67 ± 18.074 | 83.53 ± 18.202 | -2.14 ± 4.605 | -2.00 (-16.0, 17.1) |

| End of Period^{b} | | | | | |
|---|---|---|---|---|---|
| APR 20 BID | 943 | 85.81 ± 21.154 | 84.65 ± 20.541 | -1.16 ± 4.255 | -0.50 (-44.3, 14.0) |
| APR 30 BID | 944 | 85.06 ± 20.091 | 84.09 ± 20.309 | -0.96 ± 5.534 | -0.95 (23.0, 81.3) |

| | | | | | |
|---|---|---|---|---|---|
| APR 20/30 BID = apremilast 20/30 mg twice daily; max = maximum; min = minimum; PsA = psoriatic arthritis. a At the end of period, n = number of subjects with a baseline value and at least 1 postbaseline value. b The last postbaseline value during apremilast exposure, including data up to 28 days after the last dose of investigational product but excluding the observational follow-up visit. Note: Data collected later than 28 days after the last dose of investigational product are excluded. Data in the randomized withdrawal phase (Weeks 32 to 52) from 28 days after initiating placebo until apremilast was resumed are excluded. | | | | | |

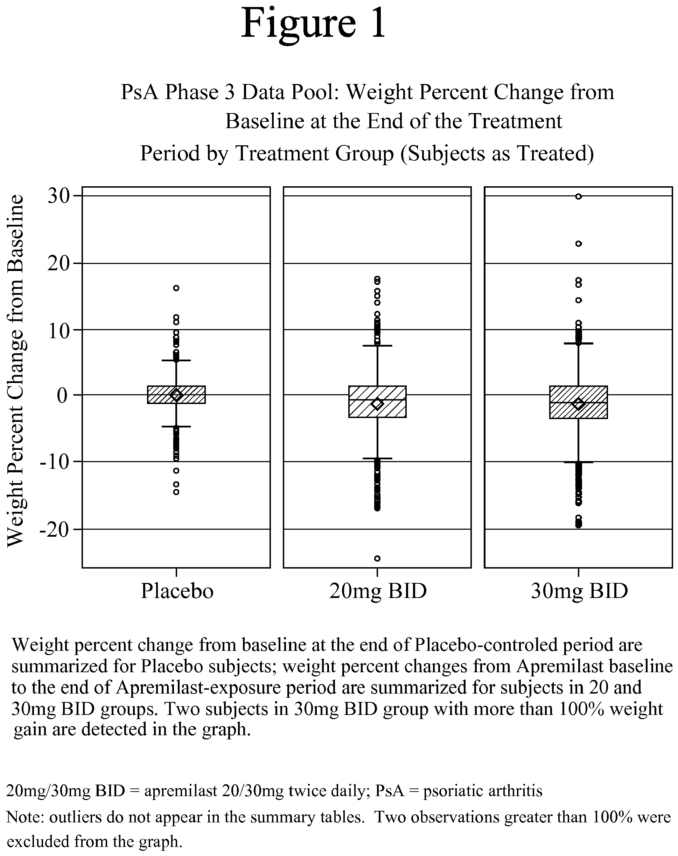

A graphical presentation of weight percent change from baseline at the end of the treatment period for the subjects as treated population in the PsA Phase 3 Data Pool is provided in Figure 1.

A summary of a categorical analysis of weight percent change from baseline at end of period during the Treatment Duration Period Weeks 0 to 16 and the Apremilast-exposure Period for Apremilast subjects as treated population is provided in Table 3. The majority of subjects receiving apremilast maintained their body weight within 0% and 5% of baseline regardless of duration of exposure. However, during Weeks 0 to 16, weight change of ≥ -10% to < -5% was observed in a higher percentage of subjects who received APR 20 BID (4.0%) or APR 30 BID (4.1%), compared with those who received placebo (2.0%). Observed weight loss of greater than 10% during Weeks 0 to 16 occurred in 1 (0.2%) subject in the placebo group, 6 (0.7%) subjects in the APR 20 BID group, and 7 (0.8%) subjects in the APR 30 BID group.

For the Apremilast-exposure Period, weight loss of greater than 10% occurred in 42 (4.5%) subjects in the APR 20 BID group and 36 (3.8%) subjects in the APR 30 BID group.

**Table 3: PsA Phase 3 Data Pool: Summary of Weight Percent Change From Baseline at the End of Period (Subjects as Treated)**

| **Weight % Change Category** | **Placebo^{a}** | | **APR 20 BID** | | **APR 30 BID** |
|---|---|---|---|---|---|
| | **Weeks 0-16^{b}** | **Weeks 0-16^{b}** | **Apremilast-exposure Period^{c}** | **Weeks 0-16^{b}** | **Apremilast-exposure Period^{c}** |
| | **n (%)** | **n (%)** | **n (%)** | **n (%)** | **n (%)** |
| Overall | m^{d} = 665 | m^{d} = 868 | m^{d} = 943 | m^{d} = 857 | m^{d} = 944 |
| <-20% | 0 | 0 | 1 (0.1) | 0 | 0 |
| ≥ -20% to < -10% | 1 (0.2) | 6 (0.7) | 41 (4.3) | 7 (0.8) | 36 (3.8) |
| ≥ -10% to < -5% | 13 (2.0) | 35 (4.0) | 98 (10.4) | 35 (4.1) | 120 (12.7) |
| ≥ -5% to < 0% | 247 (37.1) | 431 (49.7) | 393 (41.7) | 424 (49.5) | 387 (41.0) |
| 0% | 118 (17.7) | 122 (14.1) | 74 (7.8) | 111 (13.0) | 71 (7.5) |
| > 0% to ≤ 5% | 268 (40.3) | 260 (30.0) | 280 (29.7) | 266 (31.0) | 272 (28.8) |
| > 5% to ≤ 10% | 15 (2.3) | 13 (1.5) | 42 (4.5) | 10 (1.2) | 46 (4.9) |
| ≥ 10% to ≤ 20% | 3 (0.5) | 1 (0.1) | 14 (1.5) | 1 (0.1) | 8 (0.8) |
| > 20% | 0 | 0 | 0 | 3 (0.4) | 4 (0.4) |

| | | | | | |
|---|---|---|---|---|---|
| APR 20/30 BID = apremilast 20/30 mg twice daily; PsA = psoriaticarthritis. a Placebo data based on the Subjects as Initially Treated at Week 0 population, and are provided for purposes of comparison. Subjects who switched from placebo to APR are counted in both the placebo and APR treatment group columns. b For the Subjects as Treated population, data for the first 16 weeks of exposure are included regardless of when apremilast exposure started, ie, for subjects treated with apremilast at Week 0, data from study Weeks 0-16 are included whereas for subjects who are first treated with apremilast at Week 16, data from study Weeks 16-32 are included. The end-of-period value is the last postbaseline value (only through the Placebo-controlled Period for placebo-treated subjects), including data up to 28 days after the last dose of the investigational product but excluding the observational follow-up visit. c For the Subjects as Treated population, all data while subjects were exposed to apremilast regardless of when the apremilast exposure started are included. The end-of-period value is the last postbaseline value (while subjects were receiving apremilast), including data up to 28 days after the last dose of apremilast but excluding the observational follow-up visit. Data in the Randomized Treatment Withdrawal Phase (Weeks 32 to 52) from 28 days after initiating placebo until apremilast is resumed are excluded. d m = number of subjects with a baseline value and at least 1 postbaseline value; percentages are based on m. | | | | | |

### Psoriasis (PSOR) Phase 3 Data Pool

For the Subjects as initially treated at Week 0 population in the PSOR Phase 3 Data Pool, the placebo group had a mean weight change from baseline of -0.02 kg, compared with a mean weight change from baseline of -1.45 kg in the APR 30 BID group at the end of the Placebo-controlled Period. At the end of apremilast exposure, the APR 30 BID group had mean weight change from baseline of -1.67 kg (Table 4).

**Table 4 PSOR Phase 3 Data Pool: Change in Weight From Baseline to the End of Period (Subjects as Initially Treated at Week 0)**

| **Weight, kg** | | | | **Change From Baseline (kg)** | |
|---|---|---|---|---|---|
| **Time Point Treatment Group** | **n^{a}** | **Baseline Mean ± SD** | **End of Period Mean ± SD** | **Mean ± SD** | **Media**n **(Min, Max)** |
| End of Period (PBC)^{b} | | | | | |
| Placebo | 382 | 92.12 ± 21.936 | 92.09 ± 22.133 | -0.02 ± 3.017 | 0.00 (-10.6, 13.2) |
| APR 30 BID | 784 | 92.79 ± 22.057 | 91.35 ± 21.673 | -1.45 ± 3.891 | -1.00 (-34.9,28.0) |

| End of Period (APR)^{c} | | | | | |
|---|---|---|---|---|---|
| APR 30 BID | 789 | 92.72 ± 22.027 | 91.05 ± 21.694 | -1.67 ± 5.437 | -1.00 (-39.4,22.3) |

| | | | | | |
|---|---|---|---|---|---|
| APR 30 BID = apremilast 30 mg twice daily; max = maximum; min = minimum; PBC = placebo-controlled; PSOR = psoriasis; SD = standard deviation. a At the end of period, n = number of subjects with a baseline value and at least 1 postbaseline value. b The last postbaseline value during the Placebo-controlled Period, including data up to 28 days after the last dose of investigational product but excluding the observational follow-up visit. c The last postbaseline value during apremilast exposure, including data up to 28days after the last dose of apremilast but excluding the observational follow-up visit. Note: Data collected later than 28 days after the last dose of investigational product are excluded. Data in the randomized withdrawal phase (Weeks 32 to 52) from 28 days after initiating placebo until apremilast was resumed are excluded. | | | | | |

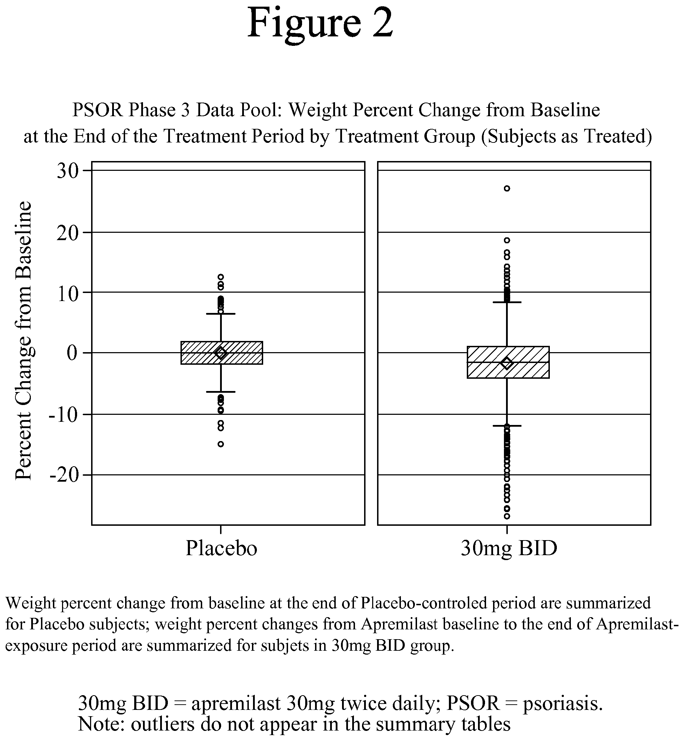

For the Apremilast subjects as treated population, mean weight change from baseline for the APR 30 BID group was -1.99 kg at Week 52 and -2.72 kg at Week 78 (Table 5). A graphical presentation of weight percent change from baseline at the end of the treatment period for the Subjects as Treated population in the PSOR Phase 3 Data Pool is provided in Figure 2.

**Table 5: PSOR Phase 3 Data Pool: Change in Weight From Baseline by Time Point (Apremilast Subjects as Treated)**

| **Weight, kg** | | | | **Change From Baseline (kg)** | |
|---|---|---|---|---|---|
| **Time Point Treatment Group** | **n^{a}** | **Baseline Mean ± SD** | **Time Point Mean ± SD** | **Mean ± SD** | **Median (Min, Max)** |
| Week 52 | | | | | |
| APR 30 BID | 657 | 92.97 ± 22.673 | 90.98 ± 22.291 | -1.99 ± 5.640 | -1.40 (-39.0, 15.6) |

| Week 78 | | | | | |
|---|---|---|---|---|---|
| APR 30 BID | 270 | 92.44 ± 22.848 | 89.72 ± 22.231 | -2.72 ± 5.792 | -2.30 (-28.0, 14.9) |

| End of Period^{b} | | | | | |
|---|---|---|---|---|---|
| APR 30 BID | 1133 | 92.48 ± 21.976 | 90.72 ± 21.669 | -1.76 ± 5.344 | -1.10 (-39.4, 22.3) |

| | | | | | |
|---|---|---|---|---|---|
| APR 30 BID = apremilast 30 mg twice daily; max = maximum; min = minimum; PSOR = psoriasis; SD = standard deviation. a At a postbaseline time point, n = number of subjects with a baseline value and a postbaseline value at the time point. At the end of period, n = number of subjects with a baseline value and at least 1 postbaseline value. b The last postbaseline value during apremilast exposure, including data up to 28 days after the last dose of apremilast but excluding the observational follow-up visit. Note: Data collected later than 28 days after the last dose of investigational product are excluded. Data in the randomized withdrawal phase (Weeks 32 to 52) from 28 days after initiating placebo until apremilast was resumed are excluded. | | | | | |

A summary of weight percent change category from baseline to the end of period by baseline weight for the PSOR Phase 3 Data Pool during Weeks 0-16, during Weeks 0-52, and during the Apremilast-exposure Period is presented in Table 6. For the subjects as initially treated at Week 0 and for the Apremilast subjects as treated populations, the majority of subjects receiving APR 30 BID maintained their body weight within 0% and 5% of baseline regardless of duration of exposure. However, during Weeks 0-16, a weight change of ≥ -10% to < -5% was observed in a larger percentage of subjects receiving APR 30 BID (11.7%) compared with placebo subjects (4.7%). Observed weight loss of greater than 10% during Weeks 0 to 16 occurred in 3 (0.8%) placebo subjects and 22 (2.0%) APR 30 BID subjects as treated. For the Apremilast-exposure Period, observed weight loss of greater than 10% occurred in 65(5.7%) APR 30 BID subjects as treated.

**Table 6: PSOR Phase 3 Data Pool: Summary of Weight Percent Change Category From Baseline at the End of Period (Subjects as Treated)**

| **Weight % Change Category** | **Placebo^{a}** | **APR 30 BID** | |
|---|---|---|---|
| | **Weeks 0-16^{b}** | **Weeks 0-16^{b}** | **Apremilast-exposure Period^{c}** |
| | **n (%)** | **n (%)** | **n (%)** |
| Overall | m^{d} = 382 | m^{d} = 1127 | m^{d} = 1133 |
| < -20% | 0 | 2 (0.2) | 9 (0.8) |
| ≥ -20% to < -10% | 3 (0.8) | 20 (1.8) | 56 (4.9) |
| ≥ -10% to < -5% | 18 (4.7) | 132 (11.7) | 162 (14.3) |
| ≥ -5% to < 0% | 155 (40.6) | 557 (49.4) | 470 (41.5) |
| 0% | 33 (8.6) | 108 (9.6) | 72 (6.4) |
| > 0% to ≤ 5% | 150 (39.3) | 277 (24.6) | 283 (25.0) |
| > 5% to ≤ 10% | 19 (5.0) | 24 (2.1) | 61 (5.4) |
| > 10% to ≤ 20% | 4 (1.0) | 6 (0.5) | 19 (1.7) |
| > 20% | 0 | 1 (0.1) | 1 (0.1) |

| | | | |
|---|---|---|---|
| APR 30 BID = apremilast 30 mg twice daily; PSOR = psoriasis. a Placebo data based on the Subjects as Initially Treated at Week 0 population, and are provided for purposes of comparison. Subjects who switched from placebo to APR are counted in both the placebo and APR treatment group columns. b For the Subjects as Treated population, data for the first 16 weeks of exposure are included regardless of when apremilast exposure started, ie, for subjects treated with apremilast at Week 0, data from study Weeks 0-16 are included whereas for subjects who are first treated with apremilast at Week 16, data from study Weeks 16-32 are included. The end-of-period value is the last postbaseline value (only through the Placebo-controlled Period for placebo-treated subjects), including data up to 28 days after the last dose of apremilast but excluding the observational follow-up visit. c For subjects as initially treated at Week 0, all data while subjects are exposed to apremilast are included. For the apremilast subjects as treated column, all data while subjects were exposed to apremilast regardless of when the apremilast exposure started are included. The end-of-period value is the last postbaseline value (while subjects were receiving apremilast), including data up to 28 days after the last dose of apremilast but excluding the observational follow-up visit. Data in the Randomized Treatment Withdrawal Phase (Weeks 32 to 52) from 28 days after initiating placebo until apremilast is resumed are excluded. d m = number of subjects with a baseline value and at least 1 postbaseline value; percentages are based on m. | | | |

### Apremilast Data Pool

In the Apremilast Data Pool for the Subjects as Initially Treated at Week 0 population, the placebo group had a mean weight change from baseline of +0.11 kg compared with a mean weight change from baseline of -1.01 kg in the APR 20 BID group and -1.21 kg in the APR 30 BID group at the end of the Placebo-controlled Period.

At the end of the Apremilast-exposure Period, the APR 20 BID and APR 30 BID groups had a mean weight change from baseline of -1.09 and -1.41 kg, respectively.

Summaries of a categorical analysis of weight percent change from baseline at end of period during the Placebo-controlled Period and during the Apremilast-exposure Period in the Apremilast Data Pool are provided in Table 7 and Table 8, respectively. For both treatment periods, the majority of subjects receiving apremilast maintained their body weight within 0% and 5% of baseline. However, during the Placebo-controlled Period, weight change of ≥ -10% to < -5% was observed in a higher percentage of subjects who received APR 20 BID (9.3%) or APR 30 BID (11.3%), compared with those who received placebo (2.8%). Observed weight loss of greater than 10% during the Placebo-controlled Period occurred in 8 (0.6%) subjects in the placebo group, 17 (1.9%) subjects in the APR 20 BID group, and 23 (1.5%) subjects in the APR 30 BID group. For the Apremilast-exposure Period, observed weight loss of greater than 10% occurred in 4.0% subjects in the APR 20 BID group and 4.9% subjects in the APR 30 BID group.

**Table 7: Apremilast Data Pool: Summary of Weight Percent Change From Baseline at the End of Period During the Placebo-controlled Period (Subjects as Initially Treated at Week 0)**

| **Weight % Change Category** | **Placebo** | **APR 20 BID** | **APR 30 BID** | **APR Total** |
|---|---|---|---|---|
| | **n (%)** | **n (%)** | **n (%)** | **n (%)** |
| Overall | m^{a} = 1274 | m^{a} = 900 | m^{a} = 1541 | m^{a} = 2591 |
| < -20% | 1 (0.1) | 1 (0.1) | 1 (0.1) | 3 (0.1) |
| ≥ -20% to < -10% | 7 (0.5) | 16 (1.8) | 22 (1.4) | 38 (1.5) |
| ≥ -10% to < -5% | 36 (2.8) | 84 (9.3) | 174 (11.3) | 263 (10.2) |
| ≥ -5% to ≤ 0% | 478 (37.5) | 393 (43.7) | 729 (47.3) | 1187 (45.8) |
| 0% | 156 (12.2) | 101 (11.2) | 159 (10.3) | 280 (10.8) |
| > 0% to ≤ 5% | 535 (42.0) | 277 (30.8) | 410 (26.6) | 741 (28.6) |
| > 5% to ≤ 10% | 52 (4.1) | 262 (2.9) | 37 (2.4) | 66 (2.5) |
| > 10% to ≤ 20% | 9 (0.7) | 2 (0.2) | 8 (0.5) | 12 (0.5) |
| > 20% | 0 | 0 | 1 (0.1) | 1 (< 0.1) |

| | | | | |
|---|---|---|---|---|
| APR 20/30 BID = apremilast 20/30 mg twice daily. a m = number of subjects with a baseline value and at least 1 postbaseline value; percentages are based on m. Note: Placebo-controlled Period includes data during the Placebo-controlled Period of each study. In studies PSA-002, PSA-003, and PSA-004, only data up to Week 16 are included for placebo-treated subjects who escaped early, whereas data up to Week 24 are included for apremilast-treated subjects in these studies. The end-of-period value is the last postbaseline value (only through the Placebo-controlled Period for placebo-treated subjects) including data up to 28 days after the last dose of investigational product but excluding the observational follow-up visit. | | | | |

**Table 8: Apremilast Data Pool: Summary of Weight Percent Change From Baseline at the End of Period During the Apremilast-exposure Period (Apremilast Subjects as Treated)**

| **Weight % Change Category** | **APR 20 BID** | **APR 30 BID** | **APR Total** |
|---|---|---|---|
| | **n (%)** | **n (%)** | **n (%)** |
| Overall | m^{a} = 1373 | m^{a} = 2268 | m^{a} = 3606 |
| < -20% | 2 (0.1) | 11 (0.5) | 14 (0.4) |
| ≥ -20% to < -10% | 53 (3.9) | 101 (4.5) | 157 (4.0) |
| ≥ -10% to < -5% | 144 (10.5) | 311 (13.7) | 466 (11.9) |
| ≥ -5% to ≤ 0% | 565 (41.2) | 923 (40.7) | 1602 (41.0) |
| 0% | 125 (9.1) | 162 (7.1) | 325 (8.3) |
| > 0% to ≤ 5% | 410 (29.9) | 610 (26.9) | 1109 (28.4) |
| > 5% to ≤ 10% | 56 (4.1) | 116 (5.1) | 179 (4.6) |
| > 10% to ≤ 20% | 18 (1.3) | 29 (1.3) | 48 (1.2) |
| > 20% | 0 | 5 (0.2) | 6 (0.2) |

| | | | |
|---|---|---|---|
| APR 20/30 BID = apremilast 20/30 mg twice daily. a m = number of subjects with a baseline value and at least 1 postbaseline value; percentages are based on m. Note: Apremilast exposure includes all data while subjects were exposed to apremilast regardless of when the apremilast exposure started. In studies PSOR-008 and PSOR-009, data collected in the randomized withdrawal phase (Weeks 32 to 52) 28 days after initiating placebo and before resuming apremilast are excluded. Duration of placebo treatment in the withdrawal phase is excluded from apremilast exposure. The end-of-period value is the last postbaseline value (while subjects were receiving apremilast), including data up to 28 days after the last dose of apremilast but excluding the observational follow-up visit. | | | |

The above data indicate that apremilast treatment provides benefit of weight loss to patients with a mean BMI of 30 or more, which is the clinical definition of obesity.

## Claims

1. A compound for use in a method of treating or managing obesity or overweight, which comprises orally administering to a patient having obesity an effective amount of the compound, wherein the compound is stereomerically pure (+)-2-[1-(3-ethoxy-4-methoxyphenyl)-2-methylsulfonylethyl]-4-acetylaminoisoindoline-1,3-dione, or a pharmaceutically acceptable polymorph, salt or solvate thereof.

2. The compound for use of claim 1, wherein the method comprises the administration of stereomerically pure (+)-2-[1-(3-ethoxy-4-methoxyphenyl)-2-methylsulfonylethyl]-4-acetylaminoisoindoline-1,3-dione once or twice daily.

3. The compound for use of claim 2, wherein the method comprises the administration of stereomerically pure (+)-2-[1-(3-ethoxy-4-methoxyphenyl)-2-methylsulfonylethyl]-4-acetylaminoisoindoline-1,3-dione in an amount of about 10 mg, 20 mg, 30 mg, or 40 mg twice daily.

4. The compound for use of claim 1, wherein the stereomerically pure (+)-2-[1-(3-ethoxy-4-methoxyphenyl)-2-methylsulfonylethyl]-4-acetylaminoisoindoline-1,3-dione comprises greater than about 90% by weight, or greater than about 95% by weight, or greater than about 96% by weight, or greater than about 97% by weight; or greater than about 98% by weight; or greater than about 99% by weight of (+) isomer based on the total weight percent of the compound.

5. The compound for use of claim 1, wherein the method comprises the administration of the stereomerically pure (+)-2-[1-(3-ethoxy-4-methoxyphenyl)-2-methylsulfonylethyl]-4-acetylaminoisoindoline-1,3-dione in a tablet form.

6. The compound for use of claim 5, wherein the tablet comprises a 10 mg, 20 mg, 30 mg, or 40 mg dose of stereomerically pure (+)-2-[1-(3-ethoxy-4-methoxyphenyl)-2-methylsulfonylethyl]-4-acetylaminoisoindoline-1,3-dione.

7. The compound for use of claim 6, wherein the tablet comprises a 10 mg dose of stereomerically pure (+)-2-[1-(3-ethoxy-4-methoxyphenyl)-2-methylsulfbnylethyl]-4-acetylaminoisoindoline-1,3-dione; or wherein the tablet comprises a 20 mg dose of stereomerically pure (+)-2-[1-(3-ethoxy-4-methoxyphenyl)-2-methylsulfonylethyl]-4-acetylaminoisoindoline-1,3-dione; or wherein the tablet comprises a 30 mg dose of stereomerically pure (+)-2-[1-(3-ethoxy-4-methoxyphenyl)-2-methylsulfonylethyl]-4-acetylaminoisoindoline-1,3-dione; or wherein the tablet comprises a 40 mg dose of stereomerically pure (+)-2-[1-(3-ethoxy-4-methoxyphenyl)-2-methylsulfonylethyl]-4-acetylaminoisoindoline-1,3-dione.

8. The compound for use of claim 6, wherein the tablet further comprises lactose monohydrate, microcrystalline cellulose, croscarmellose sodium, magnesium stearate, polyvinyl alcohol, titanium dioxide, polyethylene glycol, and talc.

9. The compound for use of claim 8, wherein the tablet further comprises iron oxide red, iron oxide red and iron oxide yellow, or iron oxide red, iron oxide yellow, and iron oxide black.

10. The compound for use of claim 1, wherein the method further comprises administering to the patient a therapeutically effective amount of one or more second active agents.

11. The compound for use of claim 10, wherein the one or more second active agents are medications for treating obesity or overweight, wherein optionally the second active agent is orlistat, lorcaserin, topiramate, phentermine, diethylpropion, phendimetrazine, benzphetamine, or a combination thereof.

12. The compound for use of claim 1, further comprising weight reduction measures of dietary habit change, exercise program, psychological support program, or a combination thereof.

13. The compound for use of claim 1, wherein the obesity is relapsed or refractory to a prior treatment.

14. The compound for use of claim 1, wherein the method further comprises administering stereomerically pure (+)-2-[1-(3-ethoxy-4-methoxyphenyl)-2-methylsulfonylethyl]-4-acetylaminoisoindoline-1,3-dione, substantially free of any salt or solvate forms of (+)-2-[1-(3-ethoxy-4-methoxyphenyl)-2-methylsulfonylethyl]-4-acetylaminoisoindoline-1,3-dione.

15. The compound for use of claim 1, wherein the method further comprises administering a pharmaceutically acceptable salt or solvate of stereomerically pure (+)-2-[1-(3-ethoxy-4-methoxyphenyl)-2-methylsulfonylethyl]-4-acetylaminoisoindoline-1,3-dione.

## Patentansprüche

1. Verbindung zur Verwendung in einem Verfahren zur Behandlung von oder zum Umgang mit Adipositas oder Übergewicht, das die orale Verabreichung einer wirksamen Menge der Verbindung an einen Adipositas leidenden Patienten umfasst, wobei die Verbindung stereomerenreines (+)-2-[1-(3-Ethoxy-4-methoxyphenyl)-2-methylsulfonylethyl]-4-acetylaminoisoindolin-1,3-dion oder ein pharmazeutisch annehmbares Polymorph, Salz oder Solvat davon ist.

2. Verbindung zur Verwendung nach Anspruch 1, wobei das Verfahren die Verabreichung von stereomerenreinem (+)-2-[1-(3-Ethoxy-4-methoxyphenyl)-2-methylsulfonylethyl]-4-acetylaminoisoindolin-1,3-dion einmal oder zweimal täglich umfasst.

3. Verbindung zur Verwendung nach Anspruch 2, wobei das Verfahren die Verabreichung von stereomerenreinem (+)-2-[1-(3-Ethoxy-4-methoxyphenyl)-2-methylsulfonylethyl]-4-acetylaminoisoindolin-1,3-dion in einer Menge von etwa 10 mg, 20 mg, 30 mg oder 40 mg zweimal täglich umfasst.

4. Verbindung zur Verwendung nach Anspruch 1, wobei das stereomerenreine (+)-2-[1-(3-Ethoxy-4-methoxyphenyl)-2-methylsulfonylethyl]-4-acetylaminoisoindolin-1,3-dion mehr als etwa 90 Gew.-% oder mehr als etwa 95 Gew.-% oder mehr als etwa 96 Gew.-% oder mehr als etwa 97 Gew.-% oder mehr als etwa 98 Gew.-% oder mehr als etwa 99 Gew.-% des (+)-Isomers auf Grundlage des gesamten Gewichtsprozentsatzes der Verbindung umfasst.

5. Verbindung zur Verwendung nach Anspruch 1, wobei das Verfahren die Verabreichung des stereomerenreinen (+) -2 - [1 - (3-Ethoxy-4-methoxyphenyl)-2-methylsulfonylethyl]-4-acetylaminoisoindolin-1,3-dion in Form einer Tablette umfasst.

6. Verbindung zur Verwendung nach Anspruch 5, wobei die Tablette eine Dosis von 10 mg, 20 mg, 30 mg oder 40 mg von stereomerenreinem (+)-2-[1-(3-Ethoxy-4-methoxyphenyl)-2-methylsulfonylethyl]-4-acetylaminoisoindolin-1,3-dion umfasst.

7. Verbindung zur Verwendung nach Anspruch 6, wobei die Tablette eine Dosis von 10 mg von stereomerenreinem (+)-2-[1-(3-Ethoxy-4-methoxyphenyl)-2-methylsulfonylethyl]-4-acetylaminoisoindolin-1,3-dion umfasst; oder wobei die Tablette eine Dosis von 20 mg von stereomerenreinem (+)-2-[1-(3-Ethoxy-4-methoxyphenyl)-2-methylsulfonylethyl]-4-acetylaminoisoindolin-1,3-dion umfasst; oder wobei die Tablette eine Dosis von 30 mg von stereomerenreinem (+)-2-[1-(3-Ethoxy-4-methoxyphenyl)-2-methylsulfonylethyl]-4-acetylaminoisoindolin-1,3-dion umfasst; oder wobei die Tablette eine Dosis von 40 mg von stereomerenreinem (+)-2-[1-(3-Ethoxy-4-methoxyphenyl)-2-methylsulfonylethyl]-4-acetylaminoisoindolin-1,3-dion umfasst.

8. Verbindung zur Verwendung nach Anspruch 6, wobei die Tablette ferner Lactose-Monohydrat, mikrokristalline Cellulose, Croscarmellose-Natrium, Magnesiumstearat, Polyvinylalkohol, Titandioxid, Polyethylenglycol und Talk umfasst.

9. Verbindung zur Verwendung nach Anspruch 8, wobei die Tablette ferner Eisenoxidrot, Eisenoxidrot und Eisenoxidgelb oder Eisenoxidrot, Eisenoxidgelb und Eisenoxidschwarz umfasst.

10. Verbindung zur Verwendung nach Anspruch 1, wobei das Verfahren ferner die Verabreichung einer therapeutisch wirksamen Menge von einem oder mehreren zweiten Wirkstoffen an den Patienten umfasst.

11. Verbindung zur Verwendung nach Anspruch 10, wobei der eine oder die mehreren zweiten Wirkstoffe Medikamente zum Behandeln von Adipositas oder Übergewicht sind, wobei gegebenenfalls der zweite Wirkstoff Orlistat, Lorcaserin, Topiramat, Phentermin, Diethylpropion, Phendimetrazin, Benzphetamin oder eine Kombination davon ist.

12. Verbindung zur Verwendung nach Anspruch 1, die ferner Gewichtsreduktionsmaßnahmen zur Änderung der Ernährungsgewohnheiten, Sportprogramm, psychologisches Unterstützungsprogramm oder eine Kombination davon umfasst.

13. Verbindung zur Verwendung nach Anspruch 1, wobei die Adipositas rückfällig oder refraktär gegenüber einer vorherigen Behandlung ist.

14. Verbindung zur Verwendung nach Anspruch 1, wobei das Verfahren ferner die Verabreichung von stereomerenreinem (+)-2-[1-(3-Ethoxy-4-methoxyphenyl)-2-methylsulfonylethyl]-4-acetylaminoisoindolin-1,3-dion umfasst, das im Wesentlichen frei ist von jeder Salz- oder Solvatform von (+)-2-[1-(3-Ethoxy-4-methoxyphenyl)-2-methylsulfonylethyl]-4-acetylaminoisoindolin-1,3-dion.

15. Verbindung zur Verwendung nach Anspruch 1, wobei das Verfahren ferner die Verabreichung eines pharmazeutisch annehmbaren Salzes oder Solvats von stereomerenreinem (+)-2-[1-(3-Ethoxy-4-methoxyphenyl)-2-methylsulfonylethyl]-4-acetylaminoisoindolin-1,3-dion umfasst.

## Revendications

1. Composé destiné à être utilisé dans une méthode de traitement ou de gestion de l'obésité ou du surpoids, qui comprend l'administration orale à un patient atteint d'obésité d'une quantité efficace du composé, dans lequel le composé est la (+)-2-[1-(3-éthoxy-4-méthoxyphényl)-2-méthylsulfonyléthyl]-4-acétylaminoisoindoline-1,3-dione stéréomériquement pure, ou un polymorphe, un sel ou un solvate pharmaceutiquement acceptable de celle-ci.

2. Composé destiné à être utilisé selon la revendication 1, dans lequel la méthode comprend l'administration de la (+)-2-[1-(3-éthoxy-4-méthoxyphényl)-2-méthylsulfonyléthyl]-4-acétylaminoisoindoline-1,3-dione stéréomériquement pure une ou deux fois par jour.

3. Composé destiné à être utilisé selon la revendication 2, dans lequel la méthode comprend l'administration de la (+)-2-[1-(3-éthoxy-4-méthoxyphényl)-2-méthylsulfonyléthyl]-4-acétylaminoisoindoline-1,3-dione stéréomériquement pure dans une quantité d'environ 10 mg, 20 mg, 30 mg ou 40 mg deux fois par jour.

4. Composé destiné à être utilisé selon la revendication 1, dans lequel la (+)-2-[1-(3-éthoxy-4-méthoxyphényl)-2-méthylsulfonyléthyl]-4-acétylaminoisoindoline-1,3-dione stéréomériquement pure comprend une quantité supérieure à environ 90 % en poids, ou supérieure à environ 95 % en poids, ou supérieure à environ 96 % en poids, ou supérieure à environ 97 % en poids ; ou supérieure à environ 98 % en poids ; ou supérieure à environ 99 % en poids d'isomère (+) sur la base du pourcentage en poids total du composé.

5. Composé destiné à être utilisé selon la revendication 1, dans lequel la méthode comprend l'administration de la (+)-2-[1-(3-éthoxy-4-méthoxyphényl)-2-méthylsulfonyléthyl]-4-acétylaminoisoindoline-1,3-dione stéréomériquement pure sous forme de comprimé.

6. Composé destiné à être utilisé selon la revendication 5, dans lequel le comprimé comprend une dose de 10 mg, 20 mg, 30 mg ou 40 mg de (+)-2-[1-(3-éthoxy-4-méthoxyphényl)-2-méthylsulfonyléthyl]-4-acétylaminoisoindoline-1,3-dione stéréomériquement pure.

7. Composé destiné à être utilisé selon la revendication 6, dans lequel le comprimé comprend une dose de 10 mg de (+)-2-[1-(3-éthoxy-4-méthoxyphényl)-2-méthylsulfonyléthyl]-4-acétylaminoisoindoline-1,3-dione stéréomériquement pure ; ou dans lequel le comprimé comprend une dose de 20 mg de (+)-2-[1-(3-éthoxy-4-méthoxyphényl)-2-méthylsulfonyléthyl]-4-acétylaminoisoindoline-1,3-dione stéréomériquement pure ; ou dans lequel le comprimé comprend une dose de 30 mg de (+)-2-[1-(3-éthoxy-4-méthoxyphényl)-2-méthylsulfonyléthyl]-4-acétylaminoisoindoline-1,3-dione stéréomériquement pure ; ou dans lequel le comprimé comprend une dose de 40 mg de (+)-2-[1-(3-éthoxy-4-méthoxyphényl)-2-méthylsulfonyléthyl]-4-acétylaminoisoindoline-1,3-dione stéréomériquement pure.

8. Composé destiné à être utilisé selon la revendication 6, dans lequel le comprimé comprend du lactose monohydraté, de la cellulose microcristalline, du croscarmellose sodique, du stéarate de magnésium, de l'alcool polyvinylique, du dioxyde de titane, du polyéthylène glycol et du talc.

9. Composé destiné à être utilisé selon la revendication 8, dans lequel le comprimé comprend en outre de l'oxyde de fer rouge, de l'oxyde de fer rouge et de l'oxyde de fer jaune, ou de l'oxyde de fer rouge, de l'oxyde de fer jaune et de l'oxyde de fer noir.

10. Composé destiné à être utilisé selon la revendication 1, dans lequel la méthode comprend en outre l'administration au patient d'une quantité thérapeutiquement efficace d'un ou de plusieurs seconds agents actifs.

11. Composé destiné à être utilisé selon la revendication 10, dans lequel les un ou plusieurs seconds agents actifs sont des médicaments destinés à traiter l'obésité ou le surpoids, dans lequel éventuellement le second agent actif est l'orlistat, la lorcasérine, le topiramate, la phentermine, le diéthylpropion, la phendimétrazine, la benzphétamine ou une combinaison de ceux-ci.

12. Composé destiné à être utilisé selon la revendication 1, comprenant en outre des mesures de réduction de poids de changement des habitudes alimentaires, de programme d'exercice, de programme de soutien psychologique ou d'une combinaison de ceux-ci.

13. Composé destiné à être utilisé selon la revendication 1, dans lequel l'obésité est une rechute ou est réfractaire à un traitement préalable.

14. Composé destiné à être utilisé selon la revendication 1, dans lequel la méthode comprend en outre l'administration de la (+)-2-[1-(3-éthoxy-4-méthoxyphényl)-2-méthylsulfonyléthyl]-4-acétylaminoisoindoline-1,3-dione stéréomériquement pure, sensiblement exempte de toutes formes salines ou solvatées de (+)-2-[1-(3-éthoxy-4-méthoxyphényl)-2-méthylsulfonyléthyl]-4-acétylaminoisoindoline-1,3-dione.

15. Composé destiné à être utilisé selon la revendication 1, dans lequel la méthode comprend en outre l'administration d'un sel ou d'un solvate pharmaceutiquement acceptable de la (+)-2-[1-(3-éthoxy-4-méthoxyphényl)-2-méthylsulfonyléthyl]-4-acétylaminoisoindoline-1,3-dione stéréomériquement pure.
